# EUROPEAN PATENT APPLICATION

(11) **EP 4 089 117 A1**
(43) Date of publication of application: **16.11.2022**
(21) Application number: 21746991.5
(22) Date of filing: 29.01.2021
(51) Int. Cl.: C07K 16/32

(54) **PH-SENSITIVE FC VARIANT**

(30) Priority: 29.01.2020 KR 20200010336; 09.11.2020 KR 20200148372; 09.11.2020 KR 20200148373
(71) Applicant: Korea University Research and Business Foundation, Seoul 02841 (KR)
(72) Inventor: JUNG, Sang-Taek, Seongnam-si Gyeonggi-do 13562 (KR); KO, Sang-Hwan, Seoul 02709 (KR); JO, Mi-Gyeong, Seoul 02717 (KR)
(74) Representative: BCKIP Part mbB
(86) International application number: PCT/KR2021/001237
(87) International publication number: WO 2021/154046

(57) **Abstract**

The present disclosure pertains to an Fc variant having an improved half-life due to pH-dependent association and dissociation from FcRn. The Fc variant has a maximized blood half-life and exhibits a pH-sensitive FcRn association and dissociation ability superior to those of conventional blood half-life-improved Fc variants. Thus, the Fc variant can bind to numerous peptide drug therapeutics having a low half-life and retention time in the body, thereby enabling the peptide drug therapeutics to have an increased blood half-life and exhibit long-term drug efficacy. Accordingly, the dosage and frequency of administration of antibodies and biopharmaceuticals can be drastically reduced, and there is an effect of reducing the cost of new drug development and greatly improving the possibility of developing new drugs.

## Description

### [Technical Field]

The present disclosure relates to an Fc variant having an improved half-life due to pH-dependent association and dissociation from FcRn.

### [Background Art]

Protein therapeutic agents are widely used in clinical practice by rapidly replacing non-specific low-molecular compound therapeutic agents because they show very high specificity, low side effects, and low toxicity to disease targets. Among protein therapeutic agents currently used in clinical practice, antibody therapeutic agents and Fc-fusion protein therapeutic agents in which an antibody Fc region is fused are the mainstays.

Therapeutic antibodies are considered as one of the most effective cancer therapies because they show very high specificity for targets as compared to the existing small-molecule drugs, show low biological toxicity and few side effects, and have superior blood half-life of about 3 weeks. Indeed, big global pharmaceutical companies and research institutes are accelerating their pace in the research and development of therapeutic antibodies which effectively remove carcinogenic factors and cancer cells by specifically binding thereto. The leading pharmaceutical companies developing therapeutic antibodies include Roche, Amgen, Johnson & Johnson, Abbott, BMS, etc. Particularly, Roche is making a considerable profit with the three representative therapeutic antibodies, Herceptin, Avastin and Rituxan for anticancer treatment, which achieved about 19.5 billion dollars of sales in 2012 globally, and is leading the world antibody drug market. Johnson & Johnson, the developer of Remicade, is also growing fast in the global antibody market with increased sales. Pharmaceutical enterprises such as Abbott and BMS are also known to have many therapeutic antibodies in the last stage of development. As a consequence, biopharmaceuticals including therapeutic antibodies, which are specific for target diseases and have few side effects, are quickly taking place of small-molecule drugs that have predominated in the global pharmaceutical market.

However, antibody and protein therapeutic agents have very low absorption by the digestive organ when administered orally, and their bioavailability is very low because they are easily denatured in the digestive tract or easily degraded by proteolytic enzymes. Hence, the therapeutic agents need to be administered through intravenous or subcutaneous injection, and frequent inoculation is required to achieve a visible therapeutic effect. In the case of an antibody, which is one of the protein therapeutic agents with very good stability in blood, a high-dose therapeutic antibody (2-8 mg/weight kg) needs to be administered once every 2-3 weeks for a successful therapeutic effect. Frequent drug administration through such injections causes significant pain and discomfort in patients, and has drawbacks that may lead to local and systemic side effects such as edema and infection. To solve these drawbacks, it is necessary to dramatically improve the half-life of the therapeutic antibodies and protein therapeutic agents in the blood.

There is a report that the *in vivo* half-life of an immunoglobulin (antibody) is mediated by the binding of Fc to FcRn. The immunoglobulin Fc fragment is internalized in acidic endosomes after uptake into endothelial cells via non-specific pinocytosis. FcRn binds to the immunoglobulin at the acidic pH (<6.5) of endosomes and releases the same at the basic pH (>7.4) of the bloodstream. Thus, FcRn salvages the immunoglobulin from lysosomal degradation. When a serum immunoglobulin level decreases, more FcRn molecules are used for immunoglobulin binding to increase an amount of immunoglobulin. Conversely, when a serum immunoglobulin level rises, FcRn becomes saturated, thereby increasing the proportion of immunoglobulin that is internalized and degraded (Ghetie and Ward, Annu. Rev. Immunol. 18: 739-766, 2000). In other words, the blood half-life and persistence of an antibody largely depend on the FcRn (neonatal Fc receptor) binding, which is one of the IgG-binding ligands, with the Fc region of the antibody. The Fc region of an antibody, which serves to recruit immune leukocytes or serum complement molecules so that damaged cells such as cancer cells or infected cells may be eliminated, is a region between Cγ2 and Cγ3 domains and mediates interaction with the neonatal receptor FcRn. Its binding recycles endocytosed antibodies from the endosome to the bloodstream (Raghavan et al., 1996, Annu Rev Cell Dev Biol 12: 181-220; Ghetie et al., 2000, Annu Rev Immunol 18: 739-766). Due to the large size of the full-length molecule, this process is associated with the inhibition of kidney filtration, and has a favorable antibody serum half-life in the range of 1 to 3 weeks. In addition, the binding of Fc to FcRn plays an important role in antibody transport. Accordingly, the Fc region plays an essential role in maintaining prolonged serum persistence by circulating antibodies through intracellular trafficking and recycling mechanisms.

### [Disclosure]

### [Technical Problem]

An aspect of the present disclosure is directed to developing an antibody increasing the binding force to FcRn in the endosomes (weakly acidic pH condition) in the cells to improve the blood half-life of the antibody, and introducing a mutation which reduces the binding force to FcRn in the blood (neutral pH condition).

### [Technical Solution]

An embodiment of the present disclosure provides a pH-sensitive Fc variant.

In addition, an embodiment of the present disclosure provides a polypeptide including a pH-sensitive Fc variant.

In addition, an embodiment of the present disclosure provides an antibody including a pH-sensitive Fc variant.

In addition, an embodiment of the present disclosure provides a nucleic acid molecule encoding a pH sensitive Fc variant, polypeptide or antibody.

In addition, an embodiment of the present disclosure provides a vector including the nucleic acid molecule.

In addition, an embodiment of the present disclosure provides a host cell including the vector.

In addition, an embodiment of the present disclosure provides a protein conjugate having an increased *in vivo* half-life.

### [Advantageous Effects]

The Fc variant of an embodiment of the present disclosure has a maximized blood half-life and exhibits a pH-selective FcRn association and dissociation ability superior to those of conventional blood half-life-improved Fc variants. The Fc variant can bind to numerous peptide drug therapeutics having a low half-life and retention time in the body, thereby enabling the peptide drug therapeutics to have an increased blood half-life and exhibit long-term drug efficacy. Accordingly, the dosage and frequency of administration of antibodies and biopharmaceuticals can be drastically reduced, and there is an effect of reducing the cost of new drug development and greatly improving the possibility of developing new drugs.

### [Description of Drawings]

FIG. 1 is a diagram identifying the binding force of trastuzumab including Q311M and M428L Fc variants with FcRn at pH 6.0 and 7.4 by ELISA:
   PFc29: trastuzumab including Q311R and M428L Fc variants; and
   M: trastuzumab including Q311M and M428L Fc variants.
FIG. 2 is a diagram illustrating an SDS-PAGE gel photograph after expression purification of trastuzumab including each of the Fc variants and a schematic diagram of amino acid variant construction at an L309 position (saturation mutagenesis).
FIG. 3 is a diagram identifying the binding force of trastuzumab including Fc variants (amino acid mutation at positions Q311M, M428L and 309) with FcRn at pH 6.0 by ELISA:
   PFc29: trastuzumab including Q311R and M428L Fc variants;
   FM: trastuzumab including L309F, Q311M and M428L Fc variants;
   IM: trastuzumab including L309I, Q311M and M428L Fc variants;
   MM: trastuzumab including L309M, Q311M and M428L Fc variants;
   VM: trastuzumab including L309V, Q311M and M428L Fc variants;
   TM: trastuzumab including L309T, Q311M and M428L Fc variants;
   AM: trastuzumab including L309A, Q311M and M428L Fc variants;
   YM: trastuzumab including L309Y, Q311M and M428L Fc variants;
   HM: trastuzumab including L309H, Q311M and M428L Fc variants;
   QM: trastuzumab including L309Q, Q311M and M428L Fc variants;
   NM: trastuzumab including L309N, Q311M and M428L Fc variants;
   KM: trastuzumab including L309K, Q311M and M428L Fc variants;
   EM: trastuzumab including L309E, Q311M and M428L Fc variants;
   WM: trastuzumab including L309W, Q311M and M428L Fc variants;
   RM: trastuzumab including L309R, Q311M and M428L Fc variants; and
   SM: trastuzumab including L309S, Q311M and M428L Fc variants.

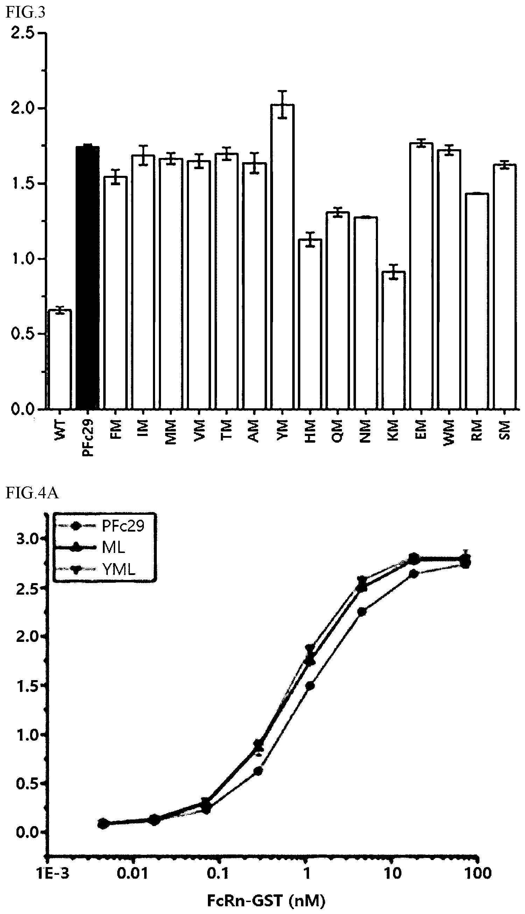

FIG. 4A is a diagram identifying the binding force of trastuzumab including Fc variants with FcRn at pH 6.0 by ELISA:
   PFc29: trastuzumab including Q311R and M428L Fc variants;
   ML: trastuzumab including Q311M and M428L Fc variants; and
   YML: trastuzumab including L309Y, Q311M and M428L Fc variants.

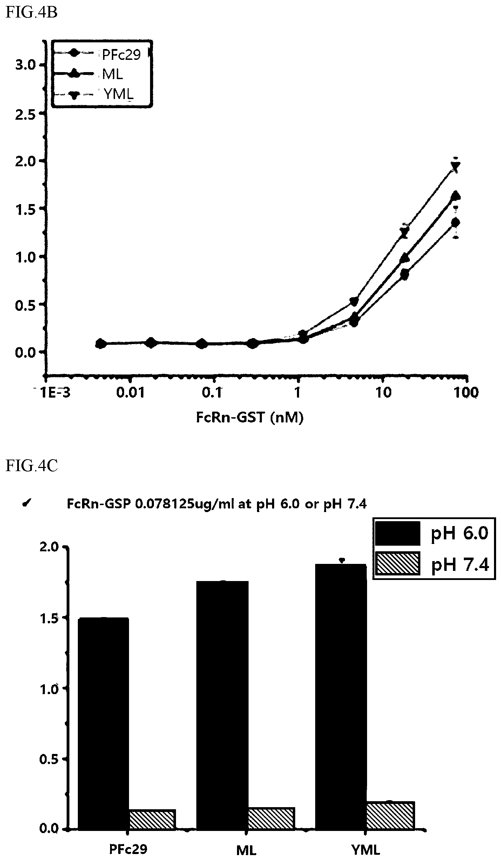

FIG. 4B is a diagram identifying the binding force of trastuzumab including Fc variants with FcRn at pH 7.4 by ELISA.
FIG. 4C is a diagram quantified by identifying the binding force of trastuzumab including Fc variants with FcRn at pH 6.0 and pH 7.4 by ELISA.

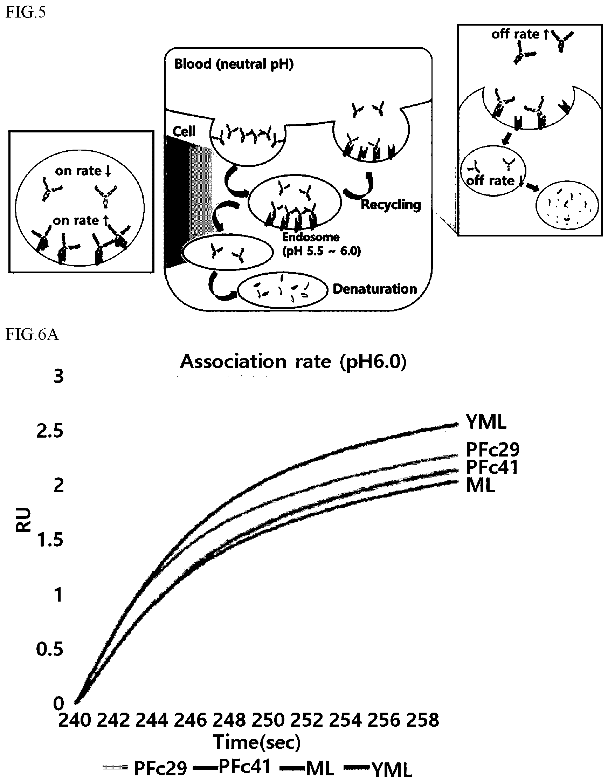

FIG. 5 is a schematic diagram of the association rate (on rate) in a weakly acidic environment and the dissociation rate (off rate) in a neutral environment.
FIG. 6A is a diagram illustrating the results of analyzing the binding rate at pH 6.0 of Fc variants:
   PFc29: trastuzumab including Q311R and M428L Fc variants;
   PFc41: trastuzumab including L309G and M428L Fc variants;
   ML: trastuzumab including Q311M and M428L Fc variants; and
   YML: trastuzumab including L309Y, Q311M and M428L Fc variants.

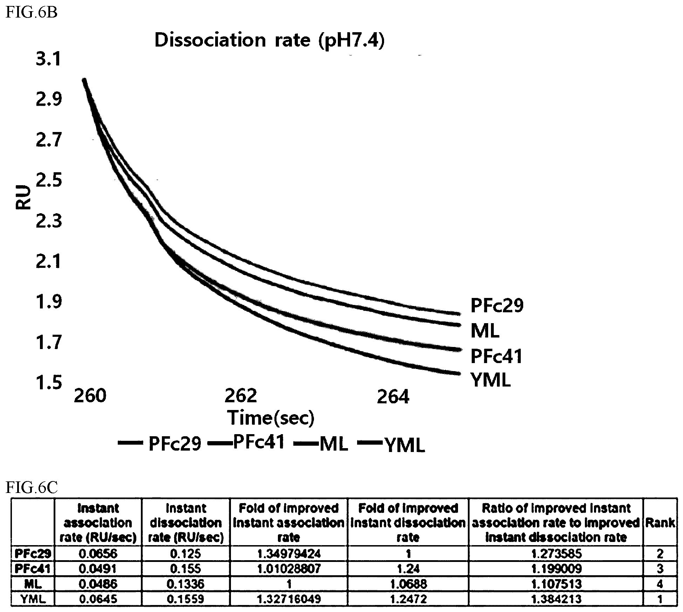

FIG. 6B is a diagram illustrating the results of analyzing the dissociation rate at pH 7.4 of Fc variants.
FIG. 6C is a diagram illustrating the results of analyzing the binding rate and dissociation rate of Fc variants.

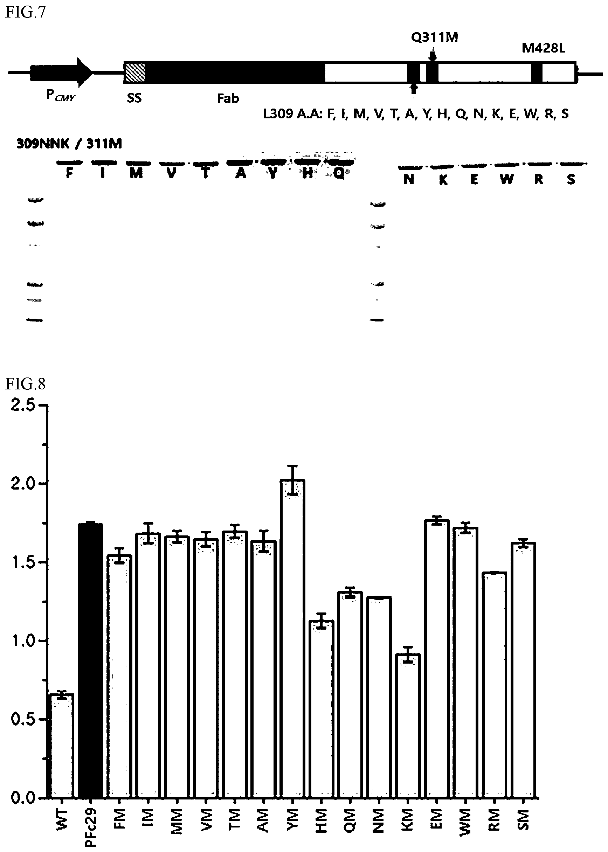

FIG. 7 is a diagram illustrating an SDS-PAGE gel photograph after expression purification of trastuzumab including a saturation mutagenesis schematic diagram at an L309 position and Fc variants.
FIG. 8 is a diagram identifying the binding force of trastuzumab including Fc variants with FcRn at pH 6.0 by ELISA.

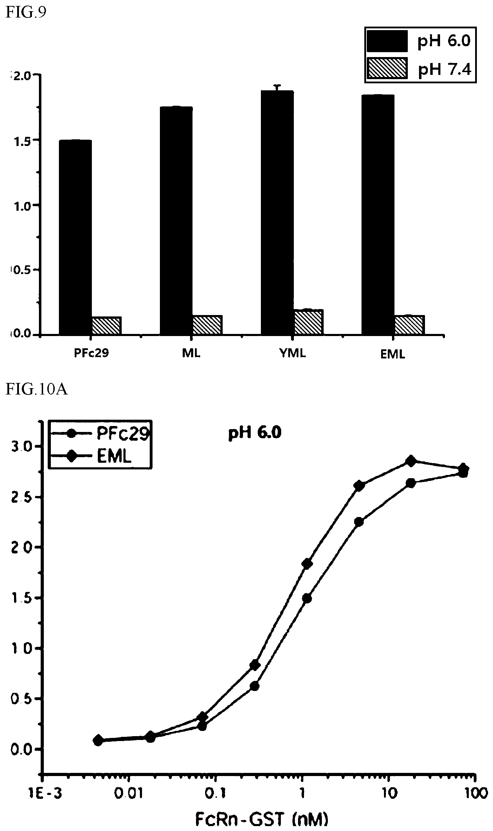

FIG. 9 is a diagram identifying the binding force of trastuzumab including Fc variants with FcRn at pH 6.0 and pH 7.4 by ELISA.
FIG. 10A is a diagram identifying the binding force of trastuzumab including EML variants with FcRn at pH 6.0 by ELISA.

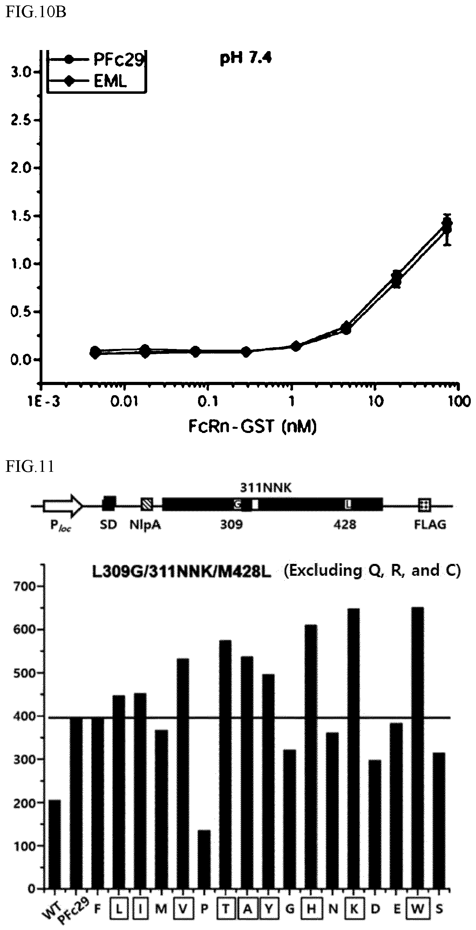

FIG. 10B is a diagram identifying the binding force of trastuzumab including EML variants with FcRn at pH 7.4 by ELISA.
FIG. 11 illustrates the results of FACS analysis for identifying the binding force to FcRn under a pH 6.0 condition of Fc variants.

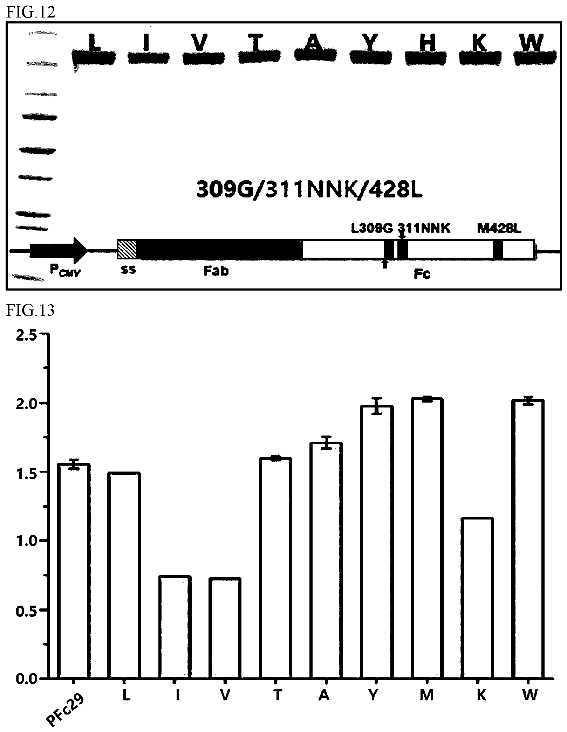

FIG. 12 illustrates an SDS-PAGE gel photograph after expression and purification of nine trastuzumab-Fc variants (Q311L, Q311I, Q311V, Q311T, Q311A, Q311Y, Q311H, Q311K or Q311W for M428L and L309G variants).
FIG. 13 illustrates the results of analysis of the binding force to FcRn at pH 6.0 of nine trastuzumab-Fc variants by ELISA (Q311L, Q311I, Q311V, Q311T, Q311A, Q311Y, Q311H, Q311K or Q311W for M428L and L309G variants).

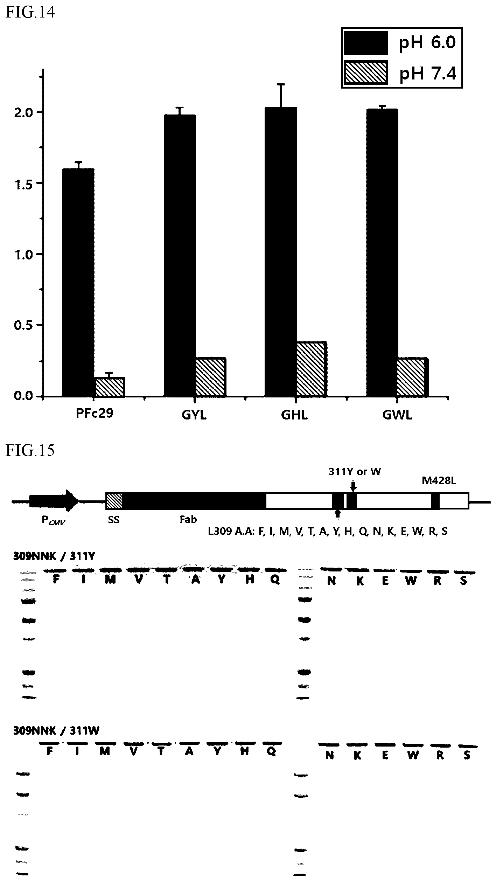

FIG. 14 illustrates the results of analyzing the binding force to FcRn at pH 6.0 and pH 7.4 of three trastuzumab-Fc variants using ELISA.
FIG. 15 is a diagram illustrating SDS-PAGE gel photographs after expression and purification of 30 trastuzumab-Fc variants.

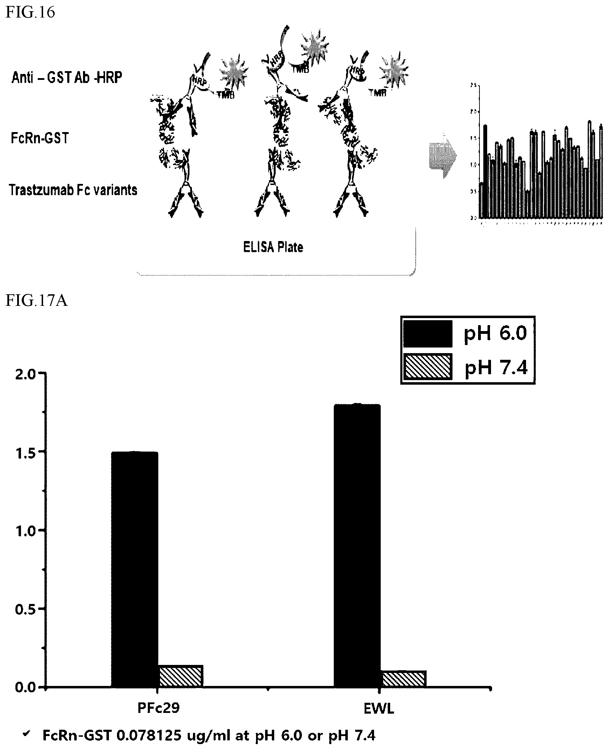

FIG. 16 illustrates the results of analyzing the binding force to FcRn at pH 6.0 of 30 trastuzumab-Fc variants by ELISA.
FIG. 17A illustrates the results of analyzing the binding force to FcRn at pH 6.0 and 7.4 of EWL (L309E/Q311W/M428L) variants using ELISA.

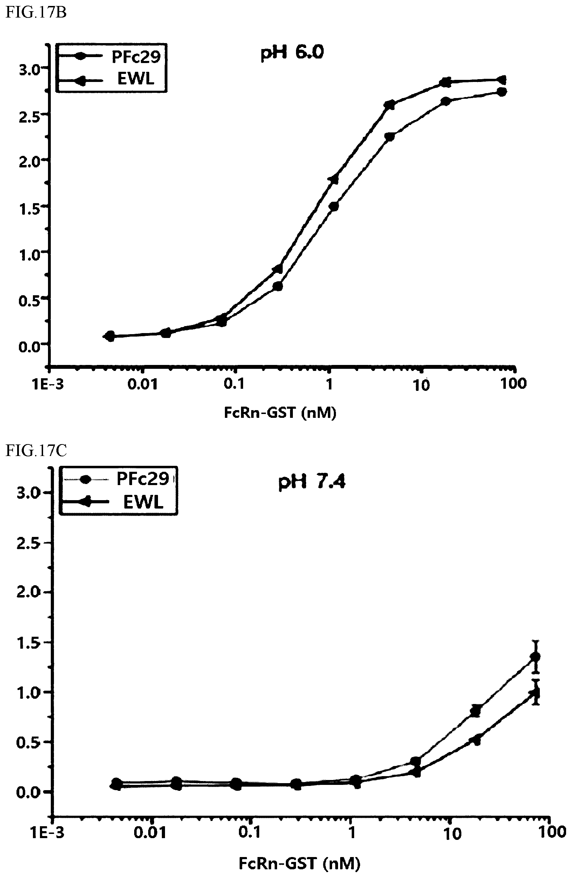

FIG. 17B illustrates the results of analyzing the binding force to FcRn at pH 6.0 of the EWL (L309E/Q311W/M428L) variants using ELISA.
FIG. 17C illustrates the results of analyzing the binding force to FcRn at pH 7.4 of EWL (L309E/Q311W/M428L) variants using ELISA.

### [Modes of the Invention]

Hereinafter, the present disclosure will be described in detail by way of embodiments of the present disclosure. However, the following embodiments are presented as examples of the present disclosure, and the present disclosure is not limited thereto. The present disclosure allows various modifications and applications within the description of the claims to be described later and the scope of equivalents interpreted therefrom.

Unless otherwise defined, all technical and scientific terms used herein have the same meaning as commonly understood by those skilled in the art to which the present disclosure pertains. Although any methods and materials similar or equivalent to those described herein may be used in the practice or testing of the present disclosure, preferred methods and materials are now described.

Throughout the disclosure of the present disclosure, not only the conventional 1-letter code and 3-letter codes for amino acids present in nature but also the 3-letter codes, such as Aib (a-aminoisobutyric acid) and Sar (N-methylglycine) generally used for other amino acids, are used. Additionally, the amino acids mentioned in abbreviation in the present disclosure are described according to the IUPAC-IUB Nomenclature.

Alanine: A, arginine: R, asparagine: N, aspartic acid: D, cysteine: C, glutamic acid: E, glutamine: Q, glycine: G, histidine: H, isoleucine: I, leucine: L, lysine: K, methionine: M, phenylalanine: F, proline: P, serine: S, threonine: T, tryptophan: W, tyrosine: Y, and valine: V

In an aspect, an embodiment of the present disclosure relates to an Fc variant including a modification of amino acid residues of L309Y according to the Kabat numbering system in an Fc region of wild-type immunoglobulin.

In an embodiment, the Fc variant of an embodiment of the present disclosure may further include a substitution of amino acid residues of M428L, Q311M, or M428L and Q311M.

In an aspect, an embodiment of the present disclosure relates to an Fc variant including a modification of amino acid residues of Q311M according to the Kabat numbering system in the Fc region of wild-type immunoglobulin.

In an embodiment, the Fc variant of an embodiment of the present disclosure may further include a substitution of amino acid residues of M428L, L309E, L309Y, M428L and L309E or M428L and L309Y.

In an aspect, an embodiment of the present disclosure relates to an Fc variant including a modification of amino acid residues of Q311W according to the Kabat numbering system in the Fc region of wild-type immunoglobulin.

In an embodiment, the Fc variant of an embodiment of the present disclosure may further include a substitution of amino acid residues of M428L, L309E, or M428L and L309E.

In an embodiment, the immunoglobulin may be IgA, IgM, IgE, IgD or IgG, or a modification thereof, and may be IgG1, IgG2, IgG3 or IgG4, preferably an anti-HER2 antibody, and more preferably trastuzumab. Papain degradation of antibodies forms two Fab fragments and one Fc fragment, and in human IgG molecules, the Fc region is produced by papain degradation of the N-terminus of Cys 226 (Deisenhofer, Biochemistry 20: 2361-2370, 1981).

In an embodiment, the Fc region of wild-type immunoglobulin may include the amino acid sequence of SEQ ID NO: 1.

In an embodiment, the Fc variant may include the amino acid sequence of SEQ ID NO: 2 (Q311M and M428L), and may include the amino acid sequence of SEQ ID NO: 3 (L309Y, Q311M and M428L).

In an embodiment, the Fc variant may include the amino acid sequence of SEQ ID NO: 4 (L309E/Q311M/M428L).

In an embodiment, the Fc variant may include the amino acid sequence of SEQ ID NO: 5 (L309E/Q311W/M428L).

In an embodiment, the Fc region of wild-type immunoglobulin may include the nucleotide sequence of SEQ ID NO: 6.

In an embodiment, the Fc variant may include the nucleotide sequence of SEQ ID NO: 7 (Q311M and M428L), and may include the nucleotide sequence of SEQ ID NO: 8 (L309Y, Q311M and M428L).

In an embodiment, the Fc variant may include the nucleotide sequence of SEQ ID NO: 9 (L309E/Q311M/M428L).

In an embodiment, the Fc variant may include the nucleotide sequence of SEQ ID NO: 10 (L309E/Q311W/M428L).

In an embodiment, the amino acid sequence of SEQ ID NO: 11 may include a wild-type trastuzumab heavy chain, and the GFNIKDTY, IYPTNGYT and SRWGGDGFYAMDY sequences each represent a CDR region. Among the amino acid sequences of SEQ ID NO: 11, the Fc region of wild-type trastuzumab corresponds to the sequence starting from the 225th.

In an embodiment, the amino acid sequence of SEQ ID NO: 12 may include a wild-type trastuzumab light chain, and QDVNTA, SAS, and QQHYTTPPT sequences each represent a CDR region.

In an embodiment, the Fc variant of the present disclosure is capable of pH-selective (dependent) association/dissociation reaction with FcRn.

In an embodiment, the Fc variant of the present disclosure may exhibit a lower binding affinity to FcRn compared to a wild-type immunoglobulin Fc region at pH 7.0 to 7.8, may be in a normal pH range of blood, and may be at pH 7.2 to 7.6. In the Fc variant of the present disclosure, the degree of dissociation from FcRn in the pH range may be the same or substantially unchanged compared to that of the wild-type Fc domain.

In an embodiment, the Fc variant of an embodiment of the present disclosure may exhibit a higher binding affinity to FcRn compared to the wild-type immunoglobulin Fc region at pH 5.6 to 6.5, may be in weakly acidic conditions in endosomes, and may be at pH 5.8 to 6.0. The pH-sensitive Fc variant of an embodiment the present disclosure has a binding affinity for FcRn in the above pH range which is higher by at least 10%, at least 20%, at least 30%, at least 40%, at least 50%, at least 60%, at least 70%, at least 80%, at least 90% or at least 100% than that of the wild-type Fc domain or by at least 2 times, at least 3 times, at least 4 times, at least 5 times, at least 6 times, at least 7 times, at least 8 times, at least 9 times, at least 10 times, at least 20 times, at least 30 times, at least 40 times, at least 50 times, at least 60 times, at least 70 times, at least 80 times, at least 90 times or at least 100 times than that of the wild-type Fc domain.

In an embodiment of the present disclosure, a variant including an amino acid mutation in an immunoglobulin Fc region of an embodiment of the present disclosure is defined according to the amino acid modification introduced into the parent immunoglobulin Fc region, and the conventional immunoglobulin numbering follows the EU index as in Kabat (Kabat et al., Sequence of proteins of immunological interest, 5th Ed., United States Public Health Service, National Institutes of Health, Bethesda, 1991).

Amino acids of SEQ ID NO: 1 to SEQ ID NO: 5 of an embodiment of the present disclosure start from number 221 of Kabat numbering. For example, Asp (D, 1D), which is the first amino acid of SEQ ID NO: 1 of an embodiment of the present disclosure, is the same as 221D by Kabat numbering, Met (M, 91M), which is the 91st amino acid of SEQ ID NO: 2, is the same as 311M by Kabat numbering, and Tyr (Y, 89Y), which is the 89th amino acid of SEQ ID NO: 3, is the same as 309Y by Kabat numbering.

As used herein, the term "trastuzumab-Fc variant" is one into which an Fc variant of an embodiment of the present disclosure have been introduced, in place of an Fc region of a wild-type trastuzumab heavy chain including the amino acid of SEQ ID NO: 11. In addition, it includes a wild-type trastuzumab light chain including the amino acid of SEQ ID NO: 12. Accordingly, in the construction of the "trastuzumab-Fc variant," an expression vector into which the Fc variant of an embodiment of the present disclosure is introduced is constructed in place of the Fc region of the wild-type trastuzumab heavy chain, and an expression vector into which the wild-type trastuzumab light chain including the amino acid of SEQ ID NO: 12 is introduced is constructed, which are then expressed by transfecting the same into animal cells.

As used herein, the term "FcRn" or "neonatal Fc receptor" means a protein that binds to the IgG antibody Fc region and is encoded at least in part by an FcRn gene. The FcRn may be derived from any organism including humans, mice, rats, rabbits, and monkeys, but is not limited thereto. As is known in the art, the functional FcRn protein includes two polypeptides, often referred to as the heavy chain and the light chain. The light chain is β-2-microglobulin and the heavy chain is encoded by the FcRn gene. Unless otherwise indicated herein, FcRn or an FcRn protein refers to the complex of FcRn heavy chain with beta-2-microglobulin.

As used herein, the term "wild-type polypeptide" means a non-modified polypeptide that is subjected to modification to generate a variant. The wild-type polypeptide is a naturally occurring polypeptide or a derivative or a manipulated one thereof. The wild-type polypeptide may refer to the polypeptide as it is, a composition including the wild-type polypeptide, or an amino acid sequence encoding the same. Accordingly, the term "wild-type immunoglobulin," as used herein, means a non-modified immunoglobulin polypeptide which generates a derivative through amino acid modifications. Interchangeably, the term "parent immunoglobulin," which means a non-modified immunoglobulin polypeptide generating a variant through amino acid modifications, may also be used.

As used herein, the term "amino acid modification" means amino acid substitution, insertion, and/or deletion, preferably substitution in a polypeptide sequence. As used herein, the term "amino acid substitution" or "substitution" means the substitution of an amino acid at a particular position in a wild-type polypeptide sequence with another amino acid. For example, an Fc variant including L309Y substitution means that leucine, which is the 309th amino acid residue in the amino acid sequence of the wild-type immunoglobulin Fc fragment, is substituted with tyrosine.

As used herein, the term "Fc variant" is meant to include modifications of one or more amino acid residues compared to a wild-type immunoglobulin Fc fragment. Preferably, the Fc variant in an embodiment of the present disclosure includes the modification of one or more amino acid residues selected from the group consisting of L309Y, Q311M and M428L (the numbering is according to the EU index described in Kabat); L309E, Q311M and M428L (the numbering is according to the EU index described in Kabat); and L309E, Q311W and M428L (the numbering is according to the EU index described in Kabat). Hence, compared to the wild-type immunoglobulin Fc fragment (region), the binding affinity for FcRn is increased under weakly acidic conditions and the binding affinity for FcRn is decreased under neutral conditions, so that the binding rate to FcRn is improved in weakly acidic intracellular endosomes and is quickly dissociated in neutral blood.

An embodiment of the present disclosure provides Fc variants having an increased binding affinity for FcRn and/or serum half-life, as compared to the corresponding wild-type immunoglobulin Fc fragment. The *in vivo* half-life of an antibody and other physiologically active molecules (that is, persistence in the serum or other tissues of a subject) is an important clinical parameter that determines administration dosage and frequency of the antibody (or any other pharmaceutical molecules). Accordingly, physiologically active molecules, including antibodies having a prolonged *in vivo* half-life, are pharmaceutically very important. The half-life of the substituted Fc variant according to an embodiment of the present disclosure may be longer by at least 10%, at least 20%, at least 30%, at least 40%, at least 50%, at least 60%, at least 70%, at least 80%, at least 90% or at least 100% than that of the wild-type Fc domain, or at least two times, at least 3 times, at least 4 times, at least 5 times, at least 6 times, at least 7 times, at least 8 times, at least 9 times or at least 10 times than that of the wild-type Fc domain.

The Fc variants according to an embodiment of the present disclosure include one or more amino acid modifications, as compared to the wild-type immunoglobulin Fc fragment (region or domain), and therefore have different amino acid sequences. The amino acid sequences of the Fc variants according to an embodiment of the present disclosure are substantially homologous to that of the wild-type immunoglobulin Fc fragment. For example, the amino acid sequences of the pH-sensitive Fc variants according to an embodiment of the present disclosure may have about 80% or higher homology, preferably about 90% or higher homology, and most preferably about 95% or higher homology than that of the wild-type immunoglobulin Fc fragment. The amino acid modification may be genetically performed by a molecular biological method or may be performed by an enzymatic or chemical method.

In an embodiment of the present disclosure, the production and purification of trastuzumab may be prepared with reference to Korean Patent Application No. 10-2017-0045142.

The Fc variants according to an embodiment of the present disclosure may be prepared by any conventional method known in the art. In an embodiment, the immunoglobulin Fc variants according to an embodiment of the present disclosure are used to create nucleic acids that encode the polypeptide sequences including particular amino acid modifications, followed by being cloned into host cells, expressed and assayed, if desired. A variety of methods are described in the literature (Molecular Cloning - A Laboratory Manual, 3rd Ed., Maniatis, Cold Spring Harbor Laboratory Press, New York, 2001; Current Protocols in Molecular Biology, John Wiley & Sons).

The nucleic acids that encode the Fc variants according to an embodiment of the present disclosure may be incorporated into an expression vector for protein expression. Expression vectors typically include a protein operably linked, that is, placed in a functional relationship, with control or regulatory sequences, selectable markers, any fusion partners, and/or additional elements. The Fc variant according to an embodiment of the present disclosure may be produced by culturing a host cell transformed with the nucleic acid, preferably an expression vector containing the nucleic acid encoding the Fc variant, under conditions appropriate so as to induce or cause the protein expression. A wide variety of appropriate host cells include, but are not limited to, mammalian cells, bacterial cells, insect cells, and yeast cells. The methods of introducing an exogenous nucleic acid into host cells are well known in the art, and will vary with the host cell used. *E. coli,* which is industrially valuable due to low production costs, may preferably be used as a host cell to produce the Fc variants according to an embodiment of the present disclosure.

Accordingly, the scope of an embodiment of the present disclosure includes a method for preparing the Fc variant, wherein the method includes: culturing the host cells, into which the nucleic acid encoding the Fc variant is introduced, under the conditions suitable for protein expression; and purifying or isolating the Fc variant expressed from the host cells.

Antibodies may be isolated or purified by various methods known in the art. Standard purification methods include chromatographic techniques, electrophoresis, immunoprecipitation, dialysis, filtration, concentration, and chromatofocusing techniques. As is well known in the art, a variety of natural proteins such as bacterial proteins A, G, and L may bind to antibodies, and thus these proteins may be used for the purification. Purification may often be enabled by using a particular fusion partner.

In an aspect, an embodiment of the present disclosure relates to a polypeptide including an Fc variant of an embodiment of the present disclosure.

In an embodiment, the polypeptide may have an increased *in vivo* half-life compared to a wild-type.

In an aspect, an embodiment of the present disclosure relates to an antibody including an Fc variant or the polypeptide.

In an embodiment, the antibody of an embodiment of the present disclosure may have an increased *in vivo* half-life compared to an antibody including a wild-type Fc region.

In an embodiment, the antibody may be a polyclonal antibody, monoclonal antibody, minibody, domain antibody, bispecific antibody, antibody mimetic, chimeric antibody, antibody conjugate, human antibody or humanized antibody, or a fragment thereof.

The antibody of an embodiment of the present disclosure may maximize the half-life of the Fc domain (region) or a polypeptide including the same through optimization of the Fc region (L309Y, Q311M and M428L; L309E, Q311M and M428L; L309E, Q311W and M428L).

In an aspect, an embodiment of the present disclosure relates to a protein conjugate having an increased *in vivo* half-life, in which the Fc variant of an embodiment of the present disclosure, a non-peptidyl polymer, and a physiologically active polypeptide are covalently linked.

In an embodiment, the Fc variant according to an embodiment of the present disclosure can be usefully used as a carrier for increasing the *in vivo* half-life of a physiologically active polypeptide such as a protein drug.

In an embodiment, the protein conjugate including the Fc variant of an embodiment of the present disclosure can be used as a long-acting drug formulation having remarkably increased *in vivo* half-life.

The non-peptidyl polymer useful in an embodiment of the present disclosure may be selected from the group consisting of biodegradable polymers such as polyethylene glycol, polypropylene glycol, a copolymer of ethylene glycol and propylene glycol, polyoxyethylated polyol, polyvinyl alcohol, polysaccharide, dextran, polyvinyl ethyl ether, PLA (polylactic acid) and PLGA (polylactic-glycolic acid), lipopolymers, chitins, hyaluronic acid and combinations thereof, and preferably polyethylene glycol. Also, their derivatives that are known in the art or that may be readily prepared using a conventional technique fall within the scope of an embodiment of the present disclosure.

The physiologically active polypeptide to be linked to the Fc variant according to an embodiment of the present disclosure may be any one without limitation, as long as it is needed to have an increased blood half-life. For example, various physiologically active polypeptides that are used for the purpose of treating or preventing human diseases, such as cytokines, interleukins, interleukin-binding proteins, enzymes, antibodies, growth factors, transcription factors, blood factors, vaccines, structural proteins, ligand proteins or receptors, cell surface antigens, and receptor antagonists, and derivatives or analogs thereof, may be used.

Examples of the physiologically active polypeptides useful in an embodiment of the present disclosure include human growth hormones, growth hormone releasing hormones, growth hormone releasing peptides, interferons and interferon receptors (e.g., interferon-alpha, - beta and -gamma, soluble type I interferon receptors), granulocyte colony-stimulating factors (G-CSF), granulocyte-macrophage colony-stimulating factors (GM-CSF), glucagon-like peptides (GLP-1), G-protein-coupled receptors, interleukins (e.g., IL-1 receptors and IL-4 receptors), enzymes (e.g., glucocerebrosidase, iduronate-2-sulfatase, alpha-galactosidase-A, agalsidase alpha, beta, alpha-L-iduronidase, butyrylcholinesterase, chitinase, glutamate decarboxylase, imiglucerase, lipase, uricase, platelet-activatingfactor acetylhydrolase, neutral endopeptidase, and myeloperoxidase), interleukin- and cytokine-binding proteins (e.g., IL-18bp and TNF-binding protein), macrophage activating factors, macrophage peptides, B-cell factors, T-cell factors, Protein A, allergy inhibitors, cell necrosis glycoproteins, immunotoxins, lymphotoxins, tumor necrosis factor, tumor suppressors, transforming growth factor, alpha-1 anti-trypsin, albumin, alpha-lactalbumin, apolipoprotein-E, erythropoietin, highly glycosylated erythropoietin, angiopoietins, hemoglobin, thrombin, thrombin receptors activating peptides, thrombomodulin, blood factor 7, blood factor 7a, blood factor 8, blood factor 9, blood factor 13, plasminogen activators, fibrin-binding peptides, urokinase, streptokinase, hirudin, Protein C, C-reactive protein, renin inhibitor, collagenase inhibitor, superoxide dismutase, leptin, platelet-derived growth factor, epithelial cell growth factor, epidermal cell growth factor, angiostatin, endostatin angiotensin, bone formation growth factor, bone formation stimulating protein, calcitonin, insulin, atriopeptin, cartilage inducing factor, elcatonin, connective tissue activating factor, tissue factor pathway inhibitor, follicle stimulating hormone, luteinizing formation hormone, luteinizing formation hormone releasing hormone, nerve growth factors (e.g., nerve growth factor, cilliary neurotrophic factor, axogenesis factor-1, brain-natriuretic peptide, glial derived neurotrophic factor, netrin, neurophil inhibitor factor, neurotrophic factor, and neuturin), parathyroid hormone, relaxin, secretin, somatomedin, insulin-like growth factor, adrenocortical hormone, glucagon, cholecystokinin, pancreatic polypeptide, gastrin releasing peptide, corticotropin releasing factor, thyroid stimulating hormone, autotaxin, lactoferrin, myostatin, receptors (e.g., TNFR(P75), TNFR(P55), IL-1 receptor, VEGF receptor, and B-cell activator receptor), receptor antagonists (e.g., IL1-Ra), cell surface antigens (e.g., CD 2, 3, 4, 5, 7, 11a, 11b, 18, 19, 20, 23, 25, 33, 38, 40, 45, and 69), monoclonal antibodies, polyclonal antibodies, antibody fragments (e.g., scFv, Fab, Fab', F(ab')2, and Fd), and virus derived vaccine antigens, but are not limited thereto. The antibody fragments may be selected from Fab, Fab', F(ab')2, Fd and scFv having an ability to bind to a particular antigen.

In an embodiment, an antibody drug may be bound to a protein conjugate including an Fc variant, and the antibody drug for cancer treatment may be trastzumab, cetuximab, bevacizumab, rituximab, basiliximab, infliximab, ipilimumab, pembrolizumab, nivolumab, atezolizumab, or avelumab.

An embodiment of the present disclosure includes a method for preparing a long-acting drug formulation by covalently linking the Fc variant to a physiologically active polypeptide via a non-peptidyl polymer.

The preparation method according to an embodiment of the present disclosure may include: covalently linking the Fc variant to the physiologically active polypeptide via the non-peptidyl polymer having a terminal reactive group; and isolating a conjugate in which the physiologically active polypeptide, the non-peptidyl polymer, and the Fc variant are covalently linked.

In an aspect, an embodiment of the present disclosure relates to a nucleic acid molecule encoding an Fc variant, polypeptide or antibody of an embodiment of the present disclosure.

The nucleic acid molecule of an embodiment of the present disclosure may be an isolated or recombinant one, and may include not only a single-strained or double-stranded DNA or RNA, but a sequence complementary thereto. The isolated nucleic acid is a nucleic acid isolated from the surrounding genetic sequence existing in the genome of an isolated individual when it is a nucleic acid isolated from a naturally formed source. A nucleic acid synthesized enzymatically or chemically from a template, e.g., a PCR product, a cDNA molecule or an oligonucleotide may also be understood as an isolated nucleic acid molecule produced from these procedures. The isolated nucleic acid molecule is a nucleic acid molecule which is in the form of a separate fragment or a component of a larger nucleic acid construct. The nucleic acid is operably linked when it is placed into a functional relationship with another nucleic acid sequence. For example, a DNA for a presence or a secretory leader is operably linked to a DNA for a polypeptide when it is expressed as a preprotein before secretion of the polypeptide, a promoter or an enhancer is operably linked to a coding sequence when it affects the transcription of the polypeptide sequence, or a ribosome binding site is operably linked to a coding sequence when it is positioned so as to facilitate translation. Generally, operably linked means that the DNA sequences being linked are contiguous and, in the case of a secretory leader, contiguous and existing in the same reading frame. However, enhancers do not have to be contiguous. The linking is accomplished by ligation at convenient restriction enzyme sites. When such sites do not exist, a synthetic oligonucleotide adapter or a linker is used in accordance with conventional practice.

In an aspect, an embodiment of the present disclosure relates to a vector including the nucleic acid molecule.

As used herein, the term "vector" means a carrier capable of inserting a nucleic acid sequence for introduction of the nucleic acid sequence into a cell which may replicate the same. The nucleic acid sequence may be exogenous or heterologous. The vector may be a plasmid, a cosmid or a virus (e.g., bacteriophage), although not being limited thereto. Those skilled in the art may construct the vector according to a standard recombination technology (Maniatis, et al., Molecular Cloning, A Laboratory Manual, Cold Spring Harbor Press, Cold Spring Harbor, N.Y., 1988; Ausubel et al., In: Current Protocols in Molecular Biology, John, Wiley & Sons, Inc, N Y, 1994; etc.).

As used herein, the term "expression vector" means a vector containing a nucleic acid sequence encoding at least a part of a transcribed gene product. In some cases, the RNA molecule is translated later into a protein, a polypeptide or a peptide. The expression vector may contain various regulatory sequences. The vector or the expression vector may contain, together with a regulatory sequence controlling transcription and translation, other nucleic acid sequences providing different functions.

In an aspect, an embodiment of the present disclosure relates to a host cell including the vector.

As used herein, the term "host cell" includes both eukaryote and prokaryote, and refers to a transformable cell of any organism that may replicate the vector or may express a gene coded by the vector. The host cell may be transfected or transformed by the vector, which means a process whereby an exogenous nucleic acid molecule is transferred or introduced into the host cell.

The host cell of an embodiment of the present disclosure is preferably a bacterial cell, CHO cell, HeLa cell, HEK293 cell, BHK-21 cell, COS7 cell, COP5 cell, A549 cell or NIH3T3 cell, but is not limited thereto.

In an aspect, an embodiment of the present disclosure relates to a method for increasing *in vivo* half-life of a physiologically active polypeptide, wherein the method includes covalently linking the Fc variant according to an embodiment of the present disclosure to the physiologically active polypeptide via a non-peptidyl polymer.

In an aspect, an embodiment of the present disclosure relates to a pharmaceutical composition for preventing or treating cancer, wherein the pharmaceutical composition includes the Fc variant, polypeptide or antibody of an embodiment of the present disclosure.

In an embodiment, there may be further provided with an immunogenic apoptosis inducer.

In an embodiment, the immunogenic apoptosis inducer may be any one or more selected from the group consisting of an anthracycline-based anticancer agent, a taxane-based anticancer agent, an anti-EGFR antibody, a BK channel agonist, a bortezomib, a cardiac glycoside, a cyclophosmide-based anticancer agent, a GADD34/PP1 inhibitor, an LV-tSMAC, a Measles virus, bleomycin, mitoxantrone, or oxaliplatin. The anthracycline-based anticancer agent may be daunorubicin, doxorubicin, epirubicin, idarubicin, pixantrone, sabarubicin, or valrubicin. The taxane-based anticancer agent may be paclitaxel or docetaxel.

By administering the pharmaceutical composition for preventing or treating cancer of an embodiment of the present disclosure together with a chemical anticancer drug (anticancer agent), it is possible to increase the cancer treatment effect of the conventional anticancer agent through the apoptosis effect of cancer cells. Co-administration may be performed simultaneously or sequentially with the anticancer agent. The examples of anticancer agents include DNA alkylating agents such as mechloethamine, chlorambucil, phenylalanine, mustard, cyclophosphamide, ifosfamide, carmustine (BCNU), lomustine (CCNU), streptozotocin, busulfan, thiotepa, cisplatin and carboplatin; anti-cancer antibiotics such as dactinomycin (actinomycin D), plicamycin, and mitomycin C; and plant alkaloids such as vincristine, vinblastine, etoposide, teniposide, topotecan and iridotecan, but are not limited thereto.

In an embodiment, the examples of cancers include leukemias and lymphomas such as acute lymphocytic leukemia, acute nonlymphocytic leukemias, chronic lymphocytic leukemia, chronic myelogenous leukemia, Hodgkin's Disease, non-Hodgkin's lymphomas, and multiple myeloma, childhood solid tumors such as brain tumors, neuroblastoma, retinoblastoma, Wilms Tumor, bone tumors, and soft-tissue sarcomas, common solid tumors of adults such as lung cancer, breast cancer, prostate cancer, urinary cancers, uterine cancers, oral cancers, pancreatic cancer, melanoma and other skin cancers, stomach cancer, ovarian cancer, brain tumors, liver cancer, laryngeal cancer, thyroid cancer, esophageal cancer, and testicular cancer. The type of cancer to be prevented or treated in an embodiment of the present disclosure is not limited.

As used herein, the term "prevention" means all actions that inhibit or delay occurrence, spread, and recurrence of cancer by administration of a pharmaceutical composition according to an embodiment of the present disclosure.

As used herein, the term "treatment" means all actions that alleviate or beneficially change the apoptosis of cancer cells or symptoms of cancer by administering a composition of an embodiment of the present disclosure. Those skilled in the art may appreciate the exact criteria of the disease on which the compositions herein have effects and determine the extent of improvement, enhancement, and treatment with reference to the data presented by the Korean Academy of Medical Sciences, etc.

The term "therapeutically effective amount" used in combination with the active ingredient in the present disclosure refers to an amount of a pharmaceutically acceptable salt of a composition effective to prevent or treat a subject disease, and the therapeutically effective amount of the composition of the present disclosure may be determined by various factors, for example, administration method, target site, the patient's condition, and the like. Therefore, the dosage when used in the human body should be determined in appropriate amounts in consideration of safety and efficacy. It is also possible to estimate the amount used in humans from the effective amount determined by animal experiments. These matters to be considered in determining the effective amount are described in, for example, Hardman and Limbird, eds., Goodman and Gilman's The Pharmacological Basis of Therapeutics, 10th ed. (2001), Pergamon Press; and E.W. Martin ed., Remington's Pharmaceutical Sciences, 18th ed. (1990), Mack Publishing Co.

The composition of an embodiment of the present disclosure is administered in a pharmaceutically effective amount. The term "pharmaceutically effective amount" used herein refers to an amount sufficient to treat the disease at a reasonable benefit/risk ratio applicable for medical treatment and an amount that does not cause side effects. The level of an effective dosage may be determined by parameters including a health status of the patient, the kind of cancer, severity, the activity of a drug, sensitivity to a drug, an administration method, administration time, an administration route and a release rate, duration of treatment, formulated or co-used drugs, and other parameters well known in medical fields. The composition of the present disclosure may be administered as an individual therapeutic agent or in combination with other therapeutic agents. It may be administered sequentially or simultaneously with a conventional therapeutic agent or administered in a single or multiple dose regime. In consideration of all of the above factors, it is important to administer such a dose as to obtain a maximum effect with a minimal amount without a side effect and the dose may be easily determined by those skilled in the art.

The pharmaceutical composition of the present disclosure may further include a pharmaceutically acceptable additive, which is exemplified by starch, gelatinized starch, microcrystalline cellulose, milk sugar, povidone, colloidal silicon dioxide, calcium hydrogen phosphate, lactose, mannitol, taffy, Arabia rubber, pregelatinized starch, corn starch, cellulose powder, hydroxypropyl cellulose, Opadry, sodium starch glycolate, carnauba wax, synthetic aluminum silicate, stearic acid, magnesium stearate, aluminum stearate, calcium stearate, white sugar, dextrose, sorbitol, talc, etc. The pharmaceutically acceptable additive of the present disclosure is preferably added to the composition in an amount of 0.1 to 90 parts by weight but is not limited thereto.

The compositions of an embodiment of the present disclosure may include carriers, diluents, excipients, or a combination of two or more thereof commonly used in biological formulations. The pharmaceutically acceptable carrier is not particularly limited as long as it is suitable for the delivery of the composition to the living body. For example, compounds disclosed in Merck Index, 13th ed., Merck & Co. Inc., saline solutions, sterile water, Ringer's solution, buffered saline, dextrose solution, maltodextrin solution, glycerol, and ethanol or one or more ingredients thereof may be mixed and used. If necessary, conventional additives such as antioxidants, buffers, and bacteriostatic agents may be added. The composition may also be prepared into dosage form for injection such as aqueous solution, suspension, or emulsion, tablet, capsule, powder or pill by additionally including diluents, dispersant, surfactant, binder and lubricant. Further, the composition may be formulated into a desirable form depending on targeting disease or ingredients thereof, using the method disclosed in Remington's Pharmaceutical Science (Mack Publishing Company, Easton PA., 18th, 1990).

The composition of the present disclosure may be administered orally or parenterally (for example, intravenous, hypodermic, peritoneal or local injection). The effective dosage of the composition may be determined according to weight, age, gender, health condition, diet, administration frequency, administration method, excretion and severity of a disease. The dosage according to an embodiment of the present disclosure is 0.0001-10 mg/ml per day and preferably 0.0001-5 mg/ml per day, and administration frequency is once a day or more preferably a few times a day.

The liquid formulations for oral administration of the composition of the present disclosure include suspensions, oral liquids, emulsions, syrups and the like. In addition to water and liquid paraffin which are simple diluents commonly used, various excipients such as wetting agents, sweeteners, flavors, and preservatives may be included. Formulations for parenteral administration include sterile aqueous solutions, non-aqueous solvents, suspensions, emulsions, freeze-dried formulations, suppositories, and the like.

In an aspect, an embodiment of the present disclosure relates to a method for preparing an Fc variant having an increased *in vivo* half-life compared to a wild-type, wherein the method includes: culturing a host cell including a vector including a nucleic acid molecule encoding the Fc variant of an embodiment of the present disclosure; and recovering the Fc variant expressed by the host cell.

In an aspect, an embodiment of the present disclosure relates to a method for preparing a polypeptide including an Fc variant having an increased *in vivo* half-life compared to a wild-type, wherein the method includes: culturing a host cell including a vector including a nucleic acid molecule encoding the polypeptide of an embodiment of the present disclosure; and recovering the polypeptide expressed by the host cell.

In an aspect, an embodiment of the present disclosure relates to a method for preparing an antibody including a pH-sensitive Fc variant having an increased *in vivo* half-life compared to a wild-type, wherein the method includes: culturing a host cell including a vector including a nucleic acid molecule encoding the antibody of an embodiment of the present disclosure; and purifying the antibody expressed by the host cell.

In an embodiment, the antibody may be purified by filtration, HPLC, anion exchange or cation exchange, high-performance liquid chromatography (HPLC), affinity chromatography or a combination thereof, preferably affinity chromatography using Protein A.

### [Mode for Carrying Out Invention]

The present disclosure will be described in more detail through the following example embodiments. However, the following example embodiments are only intended to specify the contents of the present disclosure, and the present disclosure is not limited thereto.

### <Example Embodiment 1> Amino Acid Residue Modified Fc Variant of L309Y

### <1-1> Production and Purification of Fc variants (Q311M/M428L)

As seen in M252Y of YTE and M428L of LS, which are known to have a half-life improvement effect in previous studies, it was identified that Met was substituted with another amino acid in the variant with improved FcRn binding force (wild-type Fc domain amino acid sequence: SEQ ID NO: 1). For this reason, Met in the FcRn binding site of antibody Fc was determined to be an amino acid that inhibits FcRn pH-dependent binding force. In order to examine whether FcRn binding force actually inhibits binding force when introduced into PFc29 (Q311R and M428L Fc variants), trastuzumab-ML in which Fc variants (Q311M/M428L) (SEQ ID NO: 2) were introduced into the trastuzumab heavy chain (SEQ ID NO: 11) was constructed and transfected into Expi293F animal cells. Specifically, in 30 ml of Freestyle 293 expression culture medium (Gibco, 12338-018), the heavy chain gene and light chain gene of the Fc variants (Q311M/M428L) introduced with Met in Q311 were first mixed at a ratio of 1:1. Next, PEI (Polyethylenimine) (Polyscience, 23966):variant gene were mixed at a ratio of 4:1 and left at room temperature for 20 minutes, and then was mixed with the Expi293F cell line cultured and dispersed at a density of 2 × 10⁶ cells/ml one day before. After culturing for 7 days under the conditions of 37°C, 125 rpm, and 8% CO₂ in a shaking incubator, centrifugation was performed to collect only the supernatant. Thereafter, the mixture was equilibrated with 25× PBS and filtered using a 0.2 µm filter (Merck Millipore) and a bottle top filter. 500 µl of protein A resin was added to the filtered culture medium and stirred at 4°C for 16 hours. Thereafter, the resin was recovered by flowing through the column, washed with 5 ml PBS, eluted with 3 ml 100 mM glycine (pH 2.7) buffer, and neutralized using 1M Tris-HCl pH 8.0. To change the buffer, centrifugal filter units 3K (Merck Millipore) were used.

### <1-2> Identification of pH-Dependent Binding Force of Fc Variants (Q311M/M428L)

The pH-dependent binding force of trastuzumab-ML purified in Example Embodiment 1-1 to FcRn was identified by ELISA. Specifically, 50 µl each of the IgG Fc variant diluted to 4 µg/ml in 0.05 M Na₂CO₃ (pH 9.6) was immobilized on a Flat Bottom Polystyrene High Bind 96-well microplate (costar) at 4°C for 16 hours. Then, 100 µl of 4% skim milk (GenomicBase) (in 0.05% PBST pH 6.0) was blocked at room temperature for 2 hours. After washing 4 times with 180 µl of 0.05% PBST (pH 6.0), 50 µl of FcRn continuously diluted with 1% skim milk (in 0.05% PBST, pH 6.0) was dispersed into each well and reacted at room temperature for 1 hour. After the washing process, the antibody reaction was performed for 1 hour at room temperature using 50 µl of anti-GST-HRP conjugate (GEHealthcare) and washed. Thereafter, 50 µl each of 1-Step Ultra TMB-ELISA Substrate Solution (Thermo Fisher Scientific) was added to develop color, and 50 µl each of 2M H₂SO₄ was added to terminate the reaction, followed by analysis using an Epoch Microplate Spectrophotometer (BioTek).

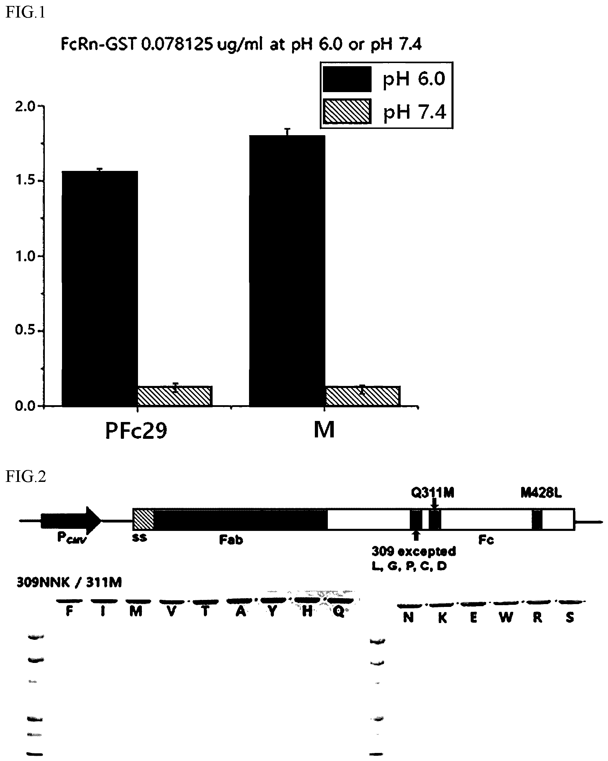

As a result, it was identified that Met, which was expected to inhibit the binding force to FcRn, rather bound better than PFc29 at pH 6.0 and showed similar binding force to PFc29 at pH 7.4. In other words, the Q311M variant bound better than PFc29 at pH 6.0 and showed a similar binding force to PFc29 at pH 7.4 (FIG. 1).

### <1-3> Searching for Fc Variants having Improved FcRn Binding Force

An attempt was made to further discover variants having improved FcRn binding force than the Fc variants having Q311M and M428L mutations selected in Example Embodiment 1-1. Specifically, 15 variants containing 15 amino acids except L, G, P, C, and D at the L309 position were constructed as in Example Embodiment 1-1, cultured in animal cells, and then purified (FIG. 2).

### <1-4> Identification of pH-Dependent Binding Force of Antibodies Including Fc Variants

In order to identify the binding force to FcRn at pH 6.0 of the variants purified in Example Embodiment 1-3, ELISA analysis was performed as in Example Embodiment 1-2. As a result, L309Y, Q311M, and M428L (trastuzumab-YML) (YM in FIG. 3, hereinafter referred to as YML, SEQ ID NO: 3) variants having the highest binding force at pH 6.0 were obtained (FIG. 3). Accordingly, the pH-dependent binding force of the selected YML variant to FcRn was measured by ELISA. As a result, it was found that the binding force to FcRn at pH 6.0 was higher than that of conventional PFc29 (Q311R and M428L Fc variants) and the ML variants selected in Example Embodiment 1-1 (Q311M and M428L Fc variants), and that a similar degree of dissociation was shown at pH 7.4. (FIGS. 4A to 4C).

### <1-5> Identification of Effect of Increasing Blood Half-Life on FcRn of Fc Variants

Since the pH-dependent binding force of Fc to FcRn is important for blood half-life improvement (*see* FIG. 5), referring to Souders *et al.* 2015, the instantaneous rate of binding in a weakly acidic environment (on rate) and the instantaneous rate of dissociation in a neutral environment (off rate) were identified in trastuzumab-ML (Q311M and M428L Fc variants) and trastuzumab-YML (L309Y, Q311M and M428L Fc variants) selected in Example Embodiments 1-1 and 1-4. As a control group thereof, conventional PFc29 (Q311R and M428L Fc variants) and PFc41 (L309G and M428L Fc variants) were used. Specifically, 40 µg/ml of His-tagged human FcRn was immobilized on an NI-NTA biosensor (Pall Fortebio) for 3 minutes. Thereafter, the baseline was set with a PBS buffer of pH 6.0, and 700 nM of each antibody Fc variant (in PBS pH 6.0) was bound for 20 seconds. In a weakly acidic environment (pH 6.0), in a state in which the antibody Fc variant was bound to FcRn, the buffer was changed to PBS at pH 7.4, dissociated for 5 seconds, and measured by biolayer interferometry assay (BLItz, Pall Fortebio). In the measured data (sensorgram), the instantaneous rate of association and instantaneous rate of dissociation were identified through the slope of the linearity section (pH 6.0, association: 2 seconds/pH 7.4, dissociation: 1 second) at each pH. Then, for comparison between the Fc variants, the slope value of each Fc variant was divided and calculated as a ratio. By scoring the average of the association rate ratio and the dissociation rate ratio, the Fc variant having the fastest association rate and dissociation rate was identified. As a result, the antibody Fc variant (YML, SEQ ID NO: 3) expected to have the greatest half-life increasing effect was selected (FIGS. 6A to 6C).

### <Example Embodiment 2> Amino Acid Residue Modified Fc Variant of Q311M

### <2-1> Production and Purification of Trastuzumab-Fc Variant Having Q311M/M428L Mutations

Upon reviewing the previous studies, it was understood that the variant with improved FcRn binding force had Met substituted with another amino acid (wild-type Fc domain amino acid sequence: SEQ ID NO: 1). For this reason, Met in the FcRn binding site of antibody Fc was determined to be an amino acid that inhibits FcRn pH-dependent binding force. Accordingly, in order to identify whether Met actually inhibits FcRn binding force when introduced into PFc29 (Q311R/M428L) discovered by the present inventors, this experiment was conducted.

More specifically, a heavy chain expression vector in which Fc variants (Q311M/M428L, SEQ ID NO: 2) were introduced into a wild-type trastuzumab heavy chain (SEQ ID NO: 11) and a wild-type trastuzumab light chain (SEQ ID NO: 12) expression vector was constructed. Thereafter, the two vectors were transfected into Expi293F animal cells. One day before transfection, Expi293F cells were subcultured at a density of 2 × 10⁶ cells/ml in 300 ml, and on the next day, the cells were transfected using PEI (Polyethylenimine, Polyscience, 23966). In 30 ml of Freestyle 293 expression culture medium (Gibco, 12338-018), the heavy and light chain genes of the variants were first mixed at a ratio of 1:1. Next, PEI: variant gene were mixed at a ratio of 4:1 and left at room temperature for 20 minutes, and then were mixed with cells subcultured on the previous day. After culturing for 7 days under the conditions of 37°C, 125 rpm, and 8% CO₂ in a shaking CO₂ incubator, centrifugation was performed to collect only the supernatant. Thereafter, the mixture was equilibrated with 25× PBS and filtered with a 0.2 µm filter (Merck Millipore) using a bottle top filter. 500 µl of protein A resin was added to the filtered culture medium, stirred at 4°C for 16 hours, and then flowed through the column. The resin was recovered, and then washed with 5 ml PBS, eluted with 3 ml 100 mM glycine pH 2.7 buffer, and then neutralized using 1M Tris-HCl pH 8.0. To change the buffer, centrifugal filter units 3K (Merck Millipore) were used.

### <2-2> ELISA Analysis of Purified Trastuzumab-Fc Variants (Q311M/M428L)

In order to measure the FcRn pH-dependent binding force with respect to the trastuzumab-ML purified in Example Embodiment 2-1, ELISA was performed.

More specifically, in order to understand the pH-dependent binding force to FcRn, 50 µl each of the IgG Fc variant diluted to 4 µg/ml in 0.05 M Na₂CO₃ (pH 9.6) was immobilized on a Flat Bottom Polystyrene High Bind 96-well microplate (costar) at 4°C for 16 hours. Then, 100 µl of 4% skim milk (GenomicBase) (in 0.05% PBST pH 6.0/pH 7.4) was blocked at room temperature for 2 hours. After washing 4 times with 180 µl of 0.05% PBST (pH 6.0/pH 7.4), 50 µl of FcRn serially diluted with 1% skim milk (in 0.05% PBST, pH 6.0/pH 7.4) was dispersed into each well and reacted at room temperature for 1 hour. After the washing process, the antibody reaction was performed for 1 hour at room temperature using 50 µl each of anti-GST-HRP conjugate (GEHealthcare) and washed. Thereafter, 50 µl each of 1-Step Ultra TMB-ELISA Substrate Solution (Thermo Fisher Scientific) was added to develop color, and 50 µl each of 2M H₂SO₄ was added to terminate the reaction, followed by analysis using an Epoch Microplate Spectrophotometer (BioTek). As a result, it was identified that Met, which was expected to inhibit the binding force to FcRn, rather bound better than PFc29 at pH 6.0 and showed similar binding force to PFc29 at pH 7.4 (FIG. 1).

In other words, contrary to the prediction before the experiment, in the case of the Q311M mutation, it was identified that the binding force was improved at pH 6.0.

### <2-3> Additional Search for Fc Variants having Improved FcRn Binding Force and Purification of Animal Cell Expression

An attempt was made to discover a variant having improved binding force than the previously selected Q311M/M428L. Specifically, 15 variants containing 15 amino acids excluding L, G, P, C, and D at the L309 position, which is known to be important for binding to FcRn, were cultured in animal cells in the same manner as in Example Embodiment 2-2 and then purified (FIG. 7).

### <2-4> ELISA Analysis of Purified Trastuzumab-Fc Variants

In order to identify the binding force of the variants prepared in Example Embodiment 2-3 at FcRn pH 6.0, ELISA was performed.

More specifically, in order to understand the pH-dependent binding force to FcRn, 50 µl each of the IgG Fc variant diluted to 4 µg/ml in 0.05 M Na₂CO₃ (pH 9.6) was immobilized on a Flat Bottom Polystyrene High Bind 96-well microplate (costar) at 4°C for 16 hours. Then, 100 µl of 4% skim milk (GenomicBase) (in 0.05% PBST pH 6.0) was blocked at room temperature for 2 hours. After washing 4 times with 180 µl of 0.05% PBST (pH 6.0), 50 µl of FcRn serially diluted with 1% skim milk (in 0.05% PBST, pH 6.0) was dispersed into each well and reacted at room temperature for 1 hour. After the washing process, the antibody reaction was performed for 1 hour at room temperature using 50 µl each of anti-GST-HRP conjugate (GEHealthcare) and washed. Thereafter, 50 µl each of 1-Step Ultra TMB-ELISA Substrate Solution (Thermo Fisher Scientific) was added to develop color, and 50 µl each of 2M H₂SO₄ was added to terminate the reaction, followed by analysis using an Epoch Microplate Spectrophotometer (BioTek). As a result, two variants of trastuzumab-YML in which a heavy chain in which Fc variants (L309Y/Q311M/M428L, SEQ ID NO: 3) were introduced into a wild-type trastuzumab heavy chain (SEQ ID NO: 11), which had higher binding force at pH 6.0 than PFc29, and a wild-type trastuzumab light chain (SEQ ID NO: 12) were bound, and trastuzumab-EML (SEQ ID NO: 4) in which a heavy chain in which Fc variants (L309E/Q311M/M428L, SEQ ID NO: 3) were introduced into a wild-type trastuzumab heavy chain (SEQ ID NO: 11) and a wild-type trastuzumab light chain (SEQ ID NO: 12) were bound, were obtained (FIG. 8).

### <2-5> Analysis of pH-Dependent FcRn Binding Force of Trastuzumab Fc Variants (L309Y/Q311M/M428L(YML), L309E/Q311M/M428L(EML))

In order to identify the FcRn pH-dependent binding force for two variants of trastuzumab-YML and trastuzumab-EML (SEQ ID NO: 4) purified in Example Embodiment 2-4, ELISA was performed.

More specifically, in order to understand the pH-dependent binding force to FcRn, 50 µl each of the IgG Fc variant diluted to 4 µg/ml in 0.05 M Na₂CO₃ (pH 9.6) was immobilized on a Flat Bottom Polystyrene High Bind 96-well microplate (costar) at 4°C for 16 hours. Then, 100 µl of 4% skim milk (GenomicBase) (in 0.05% PBST pH 6.0/pH 7.4) was blocked at room temperature for 2 hours. After washing 4 times with 180 µl of 0.05% PBST (pH 6.0/pH 7.4), 50 µl of FcRn serially diluted with 1% skim milk (in 0.05% PBST, pH 6.0/pH 7.4) was dispersed into each well and reacted at room temperature for 1 hour. After the washing process, the antibody reaction was performed for 1 hour at room temperature using 50 µl each of anti-GST-HRP conjugate (GEHealthcare) and washed. After washing, 50 µl each of 1-Step Ultra TMB-ELISA Substrate Solution (Thermo Fisher Scientific) was added to develop color, and 50 µl each of 2M H₂SO₄ was added to terminate the reaction, followed by analysis using an Epoch Microplate Spectrophotometer (BioTek).

As a result, YML showed higher binding force than PFc29 at pH 6.0 than EML, but in the case of YML, it was identified that the binding force was improved even at pH 7.4 (FIG. 9).

Accordingly, the EML variant, which showed the same binding force as PFc29 at pH 7.4 and improved binding force at pH 6.0, had improved pH-dependent binding force, which was determined to be able to improve the blood half-life by improving the recycling process of the antibody (FIGS. 10A to 10B).

### <Example Embodiment 3> Amino Acid Residue Modified Fc Variant of Q311W

### <3-1> Preparation and Analysis of 17 Amino Acid Substitution Variants at Q311 Position of PFc41 (L309G and M428L)

Based on PFc41 (L309G and M428L) and PFc29 (Q311R and M428L Fc variants) with an improved half-life in previous studies, pMopac12-NlpA-Fc variants plasmid was prepared so that L309G was fixed and the variants substituted with 17 amino acids except Q and CR at the site Q311 could be analyzed by bacterial display. The prepared plasmid was subjected to FACS analysis in order to select variants with improved binding force to human FcRn under pH 6.0 conditions using FACS. As a result, 9 variants substituted with L, I, V, T, A, Y, H, K, and W at the Q311 position with improved binding force at pH 6.0 than PFc29 discovered in the previous studies were selected (Q311L, Q311I, Q311V, Q311T, Q311A, Q311Y, Q311H, Q311K or Q311W in M428L and L309G Fc variants) (FIG. 11).

### <3-2> Production and Purification of Trastuzumab Fc Variants including L, I, V, T, A, Y, H, K, and W variants at Q311 position in PFc41 (L309G, M428L)

In order to identify the characteristics of the 9 variants selected in Example Embodiment 3-1 in an antibody format, a heavy chain expression vector in which Fc variants were introduced into a wild-type trastuzumab heavy chain (SEQ ID NO: 11) and a wild-type trastuzumab light chain (SEQ ID NO: 12) expression vector was constructed. Thereafter, Expi293F animal cells were transfected.

More specifically, one day before transfection, Expi293F cells were subcultured at a density of 2 × 10⁶ cells/ml in 30 ml, and on the next day, the cells were transfected using PEI (Polyethylenimine, Polyscience, 23966). In 3 ml of Freestyle 293 expression culture medium (Gibco, 12338-018), the heavy and light chain genes of the variants were first mixed at a ratio of 1:1. Next, PEI: variant gene were mixed at a ratio of 4:1 and left at room temperature for 20 minutes, and then were mixed with cells subcultured on the previous day. After culturing for 7 days under the conditions of 37°C, 125 rpm, and 8% CO₂ in a shaking CO₂ incubator, centrifugation was performed to collect only the supernatant. Thereafter, the mixture was equilibrated with 25× PBS and filtered with a 0.2 µm filter (Merck Millipore) using a bottle top filter. 100 µl of protein A resin was added to the filtered culture medium, stirred at 4°C for 16 hours, and then flowed through the column. The resin was recovered, and then washed with 1 ml PBS twice, eluted with 1 ml 100 mM glycine pH 2.7 buffer, and then neutralized using 1M Tris-HCl pH 8.0. To change the buffer, centrifugal filter units 30K (Merck Millipore) were used. The completed sample was identified using SDS-PAGE gel (FIG. 12).

### <3-3> ELISA Analysis of Purified Trastuzumab Fc Variants

ELISA measurement was performed to measure FcRn pH-dependent binding force for the trastuzumab-variants in which a heavy chain in which Fc variants were introduced into a wild-type trastuzumab heavy chain (SEQ ID NO: 11) and a wild-type trastuzumab light chain (SEQ ID NO: 12) were bound. First, in order to understand the binding force for FcRn under pH 6.0 conditions, 50 µl each of the IgG Fc variant diluted to 4 µg/ml in 0.05 M Na₂CO₃ (pH 9.6) was immobilized on a Flat Bottom Polystyrene High Bind 96-well microplate (costar) at 4°C for 16 hours. Then, 100 µl of 4% skim milk (GenomicBase) (in 0.05% PBST pH 6.0) was blocked at room temperature for 2 hours. After washing 4 times with 180 µl of 0.05% PBST (pH 6.0), 50 µl of FcRn serially diluted with 1% skim milk (in 0.05% PBST, pH 6.0) was dispersed into each well and reacted at room temperature for 1 hour. After the washing process, the antibody reaction was performed for 1 hour at room temperature using 50 µl each of anti-GST-HRP conjugate (GEHealthcare) and washed. 50 µl each of 1-Step Ultra TMB-ELISA Substrate Solution (Thermo Fisher Scientific) was added to develop color, and 50 µl each of 2M H₂SO₄ was added to terminate the reaction, followed by analysis using an Epoch Microplate Spectrophotometer (BioTek). As a result, the top three variants (L309G/Q311Y/M428L, L309G/Q311H/M428L, and L309G/Q311W/M428L) with improved binding force to FcRn under pH 6.0 conditions were selected (FIG. 13).

ELISA measurement was performed to measure the FcRn pH-dependent binding force of the three selected trastuzumab variants. First, in order to understand the pH-dependent binding force for FcRn, 50 µl each of the IgG Fc variant diluted to 4 µg/ml in 0.05 M Na₂CO₃ (pH 9.6) was immobilized on a Flat Bottom Polystyrene High Bind 96-well microplate (costar) at 4°C for 16 hours. Then, 100 µl of 4% skim milk (GenomicBase) (in 0.05% PBST pH 6.0/pH 7.4) was blocked at room temperature for 2 hours. After washing 4 times with 180 µl of 0.05% PBST (pH 6.0/pH 7.4), 50 µl of FcRn serially diluted with 1% skim milk (in 0.05% PBST, pH 6.0/pH 7.4) was dispersed into each well and reacted at room temperature for 1 hour. After the washing process, the antibody reaction was performed for 1 hour at room temperature using 50 µl each of anti-GST-HRP conjugate (GEHealthcare) and washed. 50 µl each of 1-Step Ultra TMB-ELISA Substrate Solution (Thermo Fisher Scientific) was added to develop color, and 50 µl each of 2M H₂SO₄ was added to terminate the reaction, followed by analysis using an Epoch Microplate Spectrophotometer (BioTek).

As a result, although the binding force to FcRn was improved over PFc29 under pH 6.0 conditions, the binding force was improved over PFc29 even at pH 7.4, so that the pH-dependent binding force was not improved (FIG. 14).

### <3-4> Saturation Mutagenesis and Expression and Purification of Animal Cells

Among the three variants selected in Example Embodiment 3-3 (L309G/Q311Y/M428L, L309G/Q311H/M428L, and L309G/Q311W/M428L), a plasmid was prepared for expressing 30 types of 15 trastuzumab variants substituted with F, I, M, V, T, A, Y, H, Q, N, K, E, W, R, and S at the L309 position while including Q311Y or W except for Q311H, which is presently known in the previous studies. Using the prepared expression vector, 30 trastuzumab mutants were cultured in animal cells in the same manner as in the previous method, then purified, and identified using SDS-PAGE gel (FIG. 15).

### <3-5> ELISA Analysis for FcRn under pH 6.0 Conditions of Trastuzumab-Fc Variant

ELISA measurement was performed to measure the binding force of 30 trastuzumab variants of Example Embodiment 3-4 at FcRn pH 6.0. First, in order to understand the pH-dependent binding force to FcRn, 50 µl each of the IgG Fc variant diluted to 4 µg/ml in 0.05 M Na₂CO₃ (pH 9.6) was immobilized on a Flat Bottom Polystyrene High Bind 96-well microplate (costar) at 4°C for 16 hours. Then, 100 µl of 4% skim milk (GenomicBase) (in 0.05% PBST pH 6.0) was blocked at room temperature for 2 hours. After washing 4 times with 180 µl of 0.05% PBST (pH 6.0), 50 µl of FcRn serially diluted with 1% skim milk (in 0.05% PBST, pH 6.0) was dispersed into each well and reacted at room temperature for 1 hour. After the washing process, the antibody reaction was performed for 1 hour at room temperature using 50 µl each of anti-GST-HRP conjugate (GEHealthcare) and washed. 50 µl each of 1-Step Ultra TMB-ELISA Substrate Solution (Thermo Fisher Scientific) was added to develop color, and 50 µl each of 2M H₂SO₄ was added to terminate the reaction, followed by analysis using an Epoch Microplate Spectrophotometer (BioTek). As a result, L309E/Q311W/M428L (EWL, SEQ ID NO: 8) variants having improved binding force than PFc29 at pH 6.0 were discovered (FIG. 16).

### <3-6> Analysis of pH-Dependent FcRn Binding Force of trastuzumab-Fc variants introduced with L309E/Q311W/M428L (EWL) mutations

ELISA was performed to measure FcRn pH-dependent binding force for the trastuzumab-EWL in which a heavy chain including EWL Fc variants (SEQ ID NO: 5) in a wild-type trastuzumab heavy chain (SEQ ID NO: 11) and a trastuzumab light chain (SEQ ID NO: 12) were bound. First, in order to understand the pH-dependent binding force to FcRn, 50 µl each of the IgG Fc variant diluted to 4 µg/ml in 0.05 M Na₂CO₃ (pH 9.6) was immobilized on a Flat Bottom Polystyrene High Bind 96-well microplate (costar) at 4°C for 16 hours. Then, 100 µl of 4% skim milk (GenomicBase) (in 0.05% PBST pH 6.0/pH 7.4) was blocked at room temperature for 2 hours. After washing 4 times with 180 µl of 0.05% PBST (pH 6.0/ pH 7.4), 50 µl of FcRn serially diluted with 1% skim milk (in 0.05% PBST, pH 6.0/pH 7.4) was dispersed into each well and reacted at room temperature for 1 hour.

After the washing process, the antibody reaction was performed for 1 hour at room temperature using 50 µl each of anti-GST-HRP conjugate (GEHealthcare) and washed. 50 µl each of 1-Step Ultra TMB-ELISA Substrate Solution (Thermo Fisher Scientific) was added to develop color, and 50 µl each of 2M H₂SO₄ was added to terminate the reaction, followed by analysis using an Epoch Microplate Spectrophotometer (BioTek). As a result, it was better bound to human FcRn under pH 6.0 conditions than PFc29 and showed lower binding force than PFc29 under pH 7.4 conditions (FIGS. 17A to 17C).

Accordingly, it may be predicted that the variant EWL has improved pH-dependent binding force, and thus can maximize the recycling process of an antibody to improve a blood half-life.

## Claims

1. An Fc variant including a modification of amino acid residues selected from the group consisting of L309Y, Q311M and Q311W according to the Kabat numbering system in an Fc region of wild-type immunoglobulin.

2. The Fc variant of claim 1, wherein the Fc variant includes a modification of amino acid residues of: L309Y and M428L; L309Y and Q311M; or L309Y, Q311M and M428L.

3. The Fc variant of claim 1, wherein the Fc variant includes a modification of amino acid residues of: 1) L309Y and Q311M; 2) L309E and Q311M; 3) Q311M and M428L; 4) L309E, Q311M and M428L; or 5) L309Y, Q311M and M428L.

4. The Fc variant of claim 1, wherein the Fc variant includes a modification of amino acid residues of: 1) L309E and Q311W; 2) Q311W and M428L; or 3) L309E, Q311W and M428L.

5. The Fc variant of claim 1, wherein the immunoglobulin is selected from the group consisting of IgA, IgM, IgE, IgD and IgG.

6. The Fc variant of claim 1, wherein the Fc variant exhibits a lower binding affinity to FcRn compared to a wild-type immunoglobulin Fc region at pH 7.0 to 7.8,

7. The Fc variant of claim 1, wherein the Fc variant exhibits a higher binding affinity to FcRn compared to a wild-type immunoglobulin Fc region at pH 5.6 to 6.5

8. A polypeptide including an Fc variant of claim 1.

9. The polypeptide of claim 8, wherein the polypeptide has an increased *in vivo* half-life compared to a wild-type.

10. An antibody including an Fc variant of claim 1.

11. The antibody of claim 10, wherein the antibody has an increased *in vivo* half-life compared to a wild-type.

12. A nucleic acid molecule encoding an Fc variant of claim 1, polypeptide of claim 8, and an antibody of claim 10.

13. A vector including a nucleic acid molecule of claim 12.

14. A host cell including a vector of claim 13.

15. A protein conjugate having an increased *in vivo* half-life, in which an Fc variant of claim 1, a non-peptidyl polymer, and a physiologically active polypeptide are covalently linked.

16. The protein conjugate of claim 15, wherein the physiologically active polypeptide is selected from the group consisting of human growth hormones, growth hormone releasing hormones, growth hormone releasing peptides, interferons, colony-stimulating factors, interleukins, interleukin water-soluble receptors, TNF water-soluble receptors, glucocerebrosidase, macrophage activating factors, macrophage peptides, B-cell factors, T-cell factors, Protein A, allergy inhibitors, cell necrosis glycoproteins, immunotoxins, lymphotoxins, tumor necrosis factor, tumor suppressors, transforming growth factor, alpha-1 anti-trypsin, albumin, apolipoprotein-E, erythropoietin, highly glycosylated erythropoietin, blood factor VII, blood factor VIII, blood factor IX, plasminogen activators, urokinase, streptokinase, Protein C, C-reactive protein, renin inhibitor, collagenase inhibitor, superoxide dismutase, leptin, platelet-derived growth factor, epidermal growth factor, bone formation growth factor, bone formation stimulating protein, calcitonin, insulin, insulin derivative, glucagon, glucagon-like peptides-1 (GLP-1), atriopeptin, cartilage inducing factor, connective tissue activating factor, follicle stimulating hormone, luteinizing formation hormone, follicle stimulating hormone releasing hormone, nerve growth factors, parathyroid hormone, relaxin, secretin, somatomedin, insulin-like growth factor, adrenocortical hormone, cholecystokinin, pancreatic polypeptide, gastrin releasing peptide, corticotropin releasing factor, thyroid stimulating hormone, receptors, receptor antagonists, cell surface antigens, monoclonal antibodies, polyclonal antibodies, antibody fragments, and virus derived vaccine antigens.
